Europäisches Patentamt
European Patent Office
Office européen des brevets

(19)

(11) Publication number: **0 186 706**
**A1**

(12)

# EUROPEAN PATENT APPLICATION

published in accordance with Art. 158(3) EPC

(21) Application number: 84902068.0

(22) Date of filing: 24.05.84

Data of the international application taken as a basis:

(86) International application number: PCT/JP 84/00267

(87) International publication number: WO 85/05360 (05.12.85 85/26)

(51) Int. Cl.[4]: **C 07 D 501/04**, C 07 D 501/06

(43) Date of publication of application: 09.07.86 Bulletin 86/28

(84) Designated Contracting States: AT BE CH DE FR GB LI NL SE

(71) Applicant: Kyoto Pharmaceutical Industries, Ltd., 38, Nishinokyo Tsukinowa-cho Nakakyo-ku, Kyoto-shi Kyoto 604 (JP)

(72) Inventor: NISHIZAWA, Susumu 1, Yamaden, Shimomisu, Yokooji Fushimi-ku, Kyoto-shi Kyoto 612 (JP)
Inventor: TAMAKI, Satoshi, 1665-24, Kojimacho, Moriyama-shi Shiga 524 (JP)
Inventor: KAKEYA, Nobuharu, 10-16, Takadai 3-chome, Nagaokakyo-shi Kyoto 617 (JP)
Inventor: KITAO, Kazuhiko 12, Sandan Osa-cho Matsugasaki, Sakyo-ku, Kyoto-shi Kyoto 606 (JP)

(74) Representative: von Kreisler, Alek, Dipl.-Chem. et al, Deichmannhaus am Hauptbahnhof, D-5000 Köln 1 (DE)

## (54) PROCESS FOR PREPARING CEPHALOSPORIN DERIVATIVES.

(57) A process for preparing cephalosporin derivatives represented by general formula (I), which comprises reacting a compound of general formula (II) or its reactive derivative with a compound of general formula (III) or eliminating an $R^1$ group from a compound of general formula (IV). In the above-mentioned formulae, $R^1$ represents formyl, t-butoxycarbonyl, 1-methyl-2-methoxycarbonylvinyl, 1-methyl-2-ethoxycarbonylvinyl, benzyloxycarbonyl, p-methoxybenzyloxycarbonyl, chloroacetyl, trityl, silyl or 2,2,2-trichloroethoxycarbonyl, and $R^2$ represents a hydrogen atom or a hydroxy group.

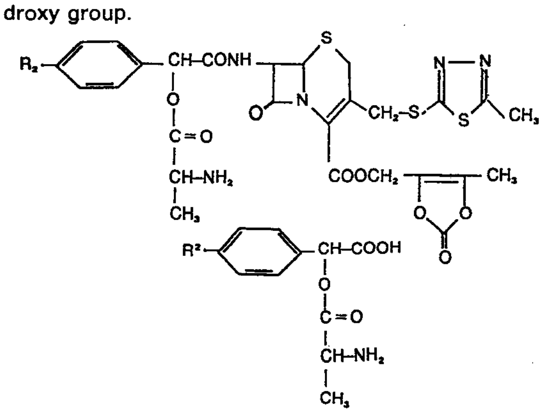

(I)

(II)

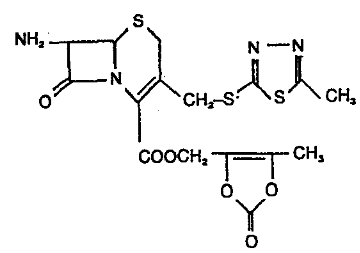

(III)

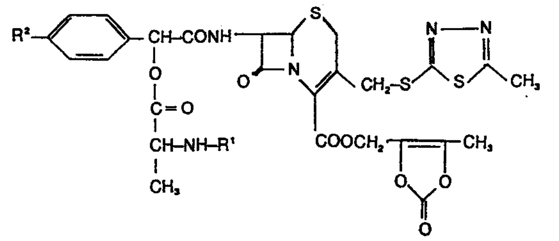

(IV)

EP 0 186 706 A1

0186706

# SPECIFICATION

## Technical Field

This invention relates to a method of producing novel cephalosporin derivatives and salts thereof.

## Background of the Invention and Prior Art

The present inventors have previously developed novel cephalosporin derivatives (I) of the general formula

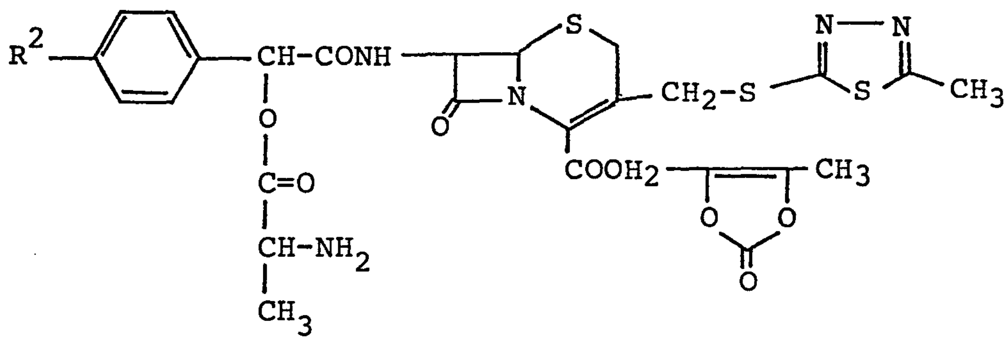

(I)

wherein $R^2$ is a hydrogen atom or a hydroxy group, and salts thereof (see Japanese Patent Application No.58-197458). Said cephalosporin derivatives (I) and salts thereof are excellent in absorbability through the digestive tract and, after absorption, they are converted rapidly to the nonester compounds corresponding to the cephalosporin derivatives (I), namely compounds of the general formula

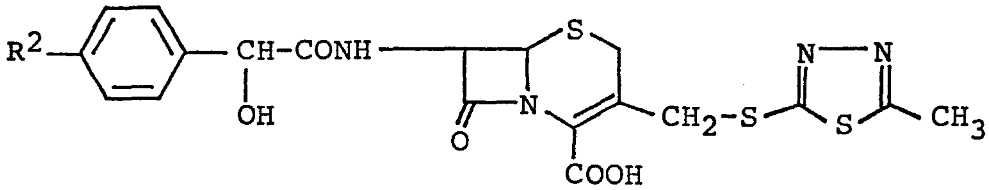

(II)

wherein $R^2$ is as defined above, as a result of in vivo enzymatic hydrolysis of the carboxylic acid ester moiety at the 4-position on the cephem ring and of the amino acid ester moiety at the α-position on the 7-position side chain. Thus, oral administration of the cephalosporin derivatives (I) results in high blood levels of the nonester compounds having excellent antibacterial activity and therefore the cephalosporin derivatives (I) and salts thereof are very useful.

The present inventors conducted intensive studies to make improvements in the method of producing said novel cephalosporin derivatives (I) and salts thereof and found that the cephalosporin derivatives (I) and salts thereof can be obtained efficiently by the method mentioned below and have now completed the present invention.

## Objects and Disclosure of the Invention

It is an object of the invention to provide a method of producing the cephalosporin derivatives (I) and salts thereof.

Another object of the invention is to provide intermediates [compounds (V) mentioned later herein] for the production of the cephalosporin derivatives (I) and salts thereof and a method of producing said intermediates.

In accordance with the invention, the cephalosporin derivatives (I) and salts thereof are produced in the following manner:

(Process 1)

A compound of the general formula

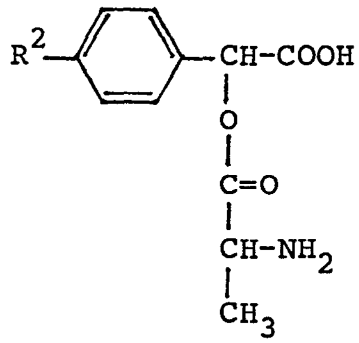

(III)

wherein $R^2$ is as defined above, is reacted with a compound of the general formula

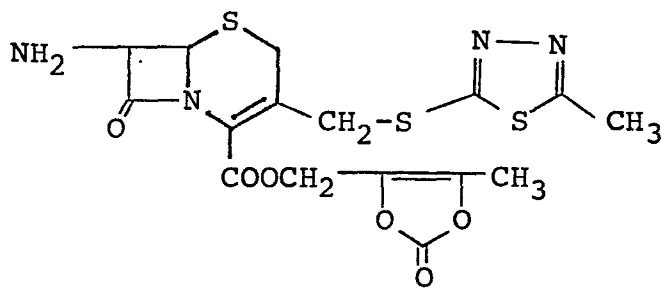

(IV)

The compound (III) is submitted to this reaction in the form of the free acid itself or an alkali metal salt thereof or a reactive derivative thereof. Thus, it is submitted to said acylation reaction in the form of the free acid itself, in the form of a salt with sodium, potassium, calcium, triethylamine or pyridine, for instance, or in the form of an acid halide (e.g. acid chloride, acid bromide), an acid anhydride, a mixed acid anhydride [e.g. with substituted phosphoric acid (e.g. dialkylphosphoric acid),

alkylcarbonic acid (e.g. monoethylcarbonic acid)], an active amide (e.g. amide with imidazole) or an ester (e.g. cyanomethyl ester, 4-nitrophenyl ester).

When the compound (III) is used in the free carboxylic acid form, an appropriate condensing agent is preferably used. The condensing agent is, for example, an N,N'-disubstituted carbodiimide, such as N,N'-dicyclohexylcarbodiimide, an azolide compound, such as N,N'-carbonyldiimidazole or N,N'-thionyldiimidazole, or the like dehydrating agent. When such condensing agent is used, the reaction presumably proceeds via a reactive derivative of the carboxylic acid.

The above reaction is generally carried out in an inert solvent. Examples of the solvent are water, acetone, dioxane, acetonitrile, chloroform, benzene, methylene chloride, ethylene chloride, tetrahydrofuran, ethyl acetate, N,N'-dimethylformamide, pyridine or the like organic solvent, or a mixture of these.

The reaction is preferably conducted at -20°C to 35°C.

(Process 2)

A compound of the general formula

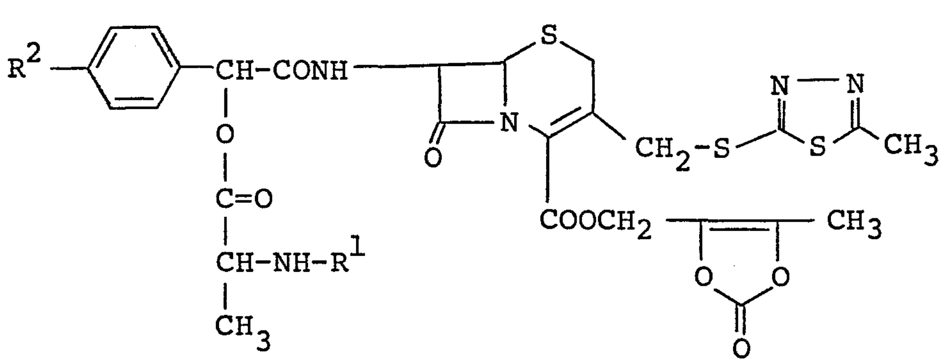

(V)

wherein $R^1$ is formyl, t-butoxycarbonyl, 1-methyl-2-methoxycarbonylvinyl, 1-methyl-2-ethoxycarbonylvinyl, benzyloxycarbonyl, p-methoxybenzyloxycarbonyl, chloroacetyl, trityl, silyl or 2,2,2-trichloroethoxycarbonyl and $R^2$ is as defined above, is subjected to amino-protecting group elimination.

Referring to $R^1$, the silyl includes trialkylsilyl, dialkylalkoxysilyl, trialkoxysilyl, etc., the alkyl preferably being a lower alkyl containing 1-4 carbon atoms, such as methyl, ethyl or propyl.

As the elimination reaction, there may be mentioned such a conventional one as hydrolysis or reduction.

(1) Hydrolysis:

The hydrolysis is preferably carried out in the presence of an acid.

The acid is, for example, an inorganic acid (e.g. hydrochloric acid, hydrobromic acid, sulfuric acid), an organic acid (e.g. formic acid, acetic acid, trifluoroacetic acid, propionic acid, methanesulfonic acid, benzenesulfonic acid, p-toluenesulfonic acid), or an acidic ion exchange resin. When an organic acid such as trifluoroacetic acid or p-toluenesulfonic acid is unsed in this reaction, the reaction is desirably conducted in the presence of a cation acceptor (e.g. anisole). The hydrolysis is generally carried out in a conventional solvent which will not disturb the reaction, such as water, methanol, ethanol,

propanol, tert-butyl alcohol, tetrahydrofuran, N,N-dimethylformamide, dioxane, acetone, methyl ethyl ketone, ethyl acetate, or a mixture of these. When the above acid is a liquid, it may serve also as a solvent.

The temperature for this hydrolysis is not critical.

(2) Reduction:

The reduction is conducted in the conventional manner of chemical reduction or catalytic reduction, among others. As an appropriate reducing agent to be used in the chemical reduction, there may be mentioned a combination of a metal (e.g. tin, zinc, iron) or metal compound (e.g. chromium chloride, chromium acetate) and an organic or inorganic acid (e.g. formic acid, acetic acid, propionic acid, trifluoroacetic acid, p-toluenesulfonic acid, hydrochloric acid, hydrobromic acid).

As appropriate examples of the catalyst for use in the catalytic reduction, there may be mentioned conventional ones such as platinum catalysts (e.g. platinum plate, platinum sponge, platinum black, colloidal platinum, platinum oxide, platinum wire), palladium catalysts (e.g. palladium sponge, palladium black, palladium oxide, palladium-on-carbon, colloidal palladium, palladium-on-barium acetate, palladium-on-barium carbonate), nickel catalysts (e.g. reduced nickel, nickel oxide, Raney nickel), cobalt catalysts (e.g. reduced cobalt, Raney cobalt), iron catalysts (e.g. reduced iron, Raney iron), and copper catalysts (e.g. reduced copper,

Raney copper, Ullmann copper).

The reduction is generally carried out in a conventional solvent which will not adversely affect the reaction, such as water, methanol, ethanol, propanol, N,N-dimethylformamide, or a mixture of these. When the acid used for the chemical reduction is a liquid, the acid may be used also as a solvent. As appropriate solvents for use in the catalytic reduction, there may be mentioned the solvents mentioned above and, furthermore, such solvents as diethyl ether, dioxane and tetrahydrofuran, and mixtures of these may also be used.

The temperature for this reduction reaction is not critical.

The method of protective group elimination is to be selected from among the methods mentioned above depending on the kind of the protective group to be removed.

The compounds (V) are novel compounds and can be produced, for example, as follows:

(Method 1)

A compound of the general formula

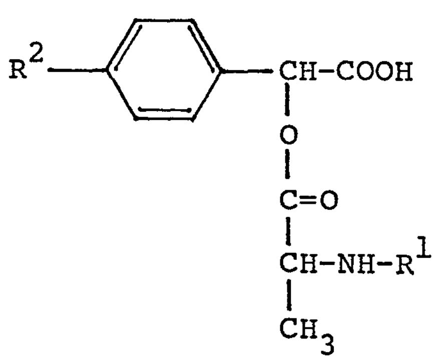

(VI)

wherein $R^1$ and $R^2$ are as defined above, or a reactive derivative thereof is reacted with a compound of the general formula

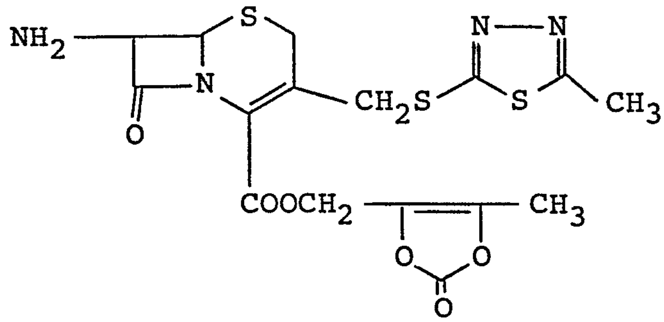

(VII)

The compound (VI) is subjected to this reaction in the form of the free carboxylic acid itself or a reactive derivative thereof. Thus, it is submitted to said acylation reaction in the free acid form or in the form of a reactive derivative such as a salt with, for example, sodium, potassium, calcium, triethylamine or pyridine, an acid halide (e.g. acid chloride, acid bromide), an acid anhydride, a mixed acid anhydride [e.g. substituted phosphoric acid (e.g. dialkylphosphoric acid), alkylcarbonic acid (monoethylcarbonic acid)], an active amide (e.g. amide with imidazole), or an ester (e.g. cyanomethyl ester, 4-nitrophenyl ester).

When the compound (VI) is used in the free acid form, an appropriate condensing agent is preferably used. As the condensing agent, there may be used, for example, an N,N'-disubstituted carbodiimide, such as N,N'-dicyclohexylcarbodiimide, an azolide compound, such as N,N'-carbonyldiimidazole

or N,N'-thionyldiimidazole, or the like dehydrating agent. When such condensing agent is used, the reaction presumably proceeds via a reactive derivative of the carboxylic acid. This reaction is generally carried out in an inert solvent. Examples of the solvent are water, acetone, dioxane, acetonitrile, chloroform, benzene, methylene chloride, ethylene chloride, tetrahydrofuran, ethyl acetate, N,N-dimethylacetamide, pyridine and other organic solvents, and mixtures of these.

This reaction is generally carried out at -30°C to 50°C, preferably at -20°C to 20°C.

The compound (VII) can be produced, for example, by reacting a compound of the general formula

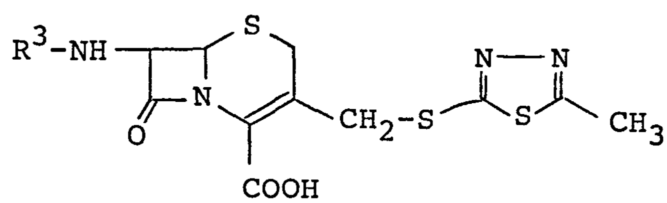

(VIII)

wherein $R^3$ is a hydrogen atom or an amino-protecting group, with a compound of the general formula

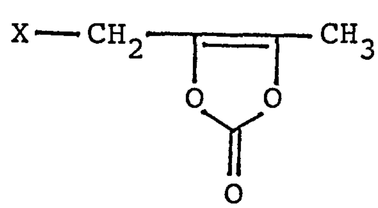

(IX)

wherein X is a group reactive with the carboxyl group (or a

reactive derivative thereof).

Referring to $R^3$ in general formula (VIII), the amino-protecting group may be a per se known amino-protecting group such as benzylcarbonyl, 2-thienylacetyl, 2-furylacetyl, D-5-amino-5-carboxyvaleryl, trityl, phthalimide, formyl, t-butoxycarbonyl, 1-methyl-2-methoxycarbonylvinyl or 1-methyl-2-ethoxycarbonylvinyl.

Referring to the general formula (IX), the group reactive with the carboxyl group (or a reactive derivative thereof) includes halogen atoms (e.g. bromo, chloro, iodo), alkylsulfonyloxy (e.g. methanesulfonyloxy), arylsulfonyloxy (e.g. p-toluenesulfonyloxy) and hydroxy, among others.

The compound (VIII) is used for the reaction in the form of the free carboxylic acid itself or a reactive derivative thereof. Thus, it is submitted to said acylation reaction either in the free acid form or in the form of a reactive derivative thereof of the same type as the reactive derivative of compound (VI).

When the compound (IX) is used in the form in which X is hydroxy, an appropriate condensing agent may be used. The same condensing agent as mentioned hereinabove may be used in this instance as well. When such condensing agent is used, the reaction presumably proceeds via a reactive derivative of the carboxylic acid.

This reaction is conducted generally at -30°C to 50°C, preferably at -20°C to 20°C.

0186706

Said protective group ($R^3$) can be eliminated by a *per se* known method.

The compound (VI) can be produced, for example, by reacting a compound of the general formula

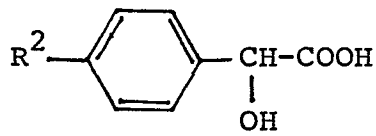

(VI-1)

wherein $R^2$ is as defined above, or a reactive derivative thereof with a compound of the general formula

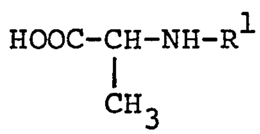

(VI-2)

wherein $R^1$ is as defined above.

(Method 2)

A compound of the general formula

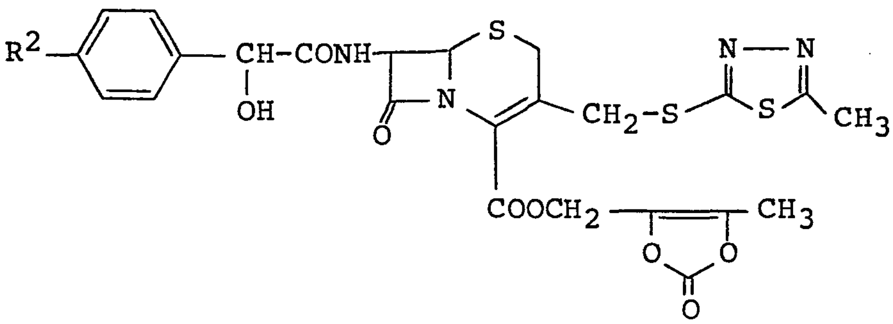

(X)

wherein $R^2$ is as defined above, or a reactive derivative

thereof is reacted with a compound of the general formula

$$R^1\text{-NH-}\underset{}{\overset{\displaystyle CH_3 \atop |}{CH}}\text{-COOH} \qquad (XI)$$

wherein $R^1$ is as defined above.

The compound (XI) is submitted to the above reaction either in the free carboxylic acid form as it is or in the form of the same reactive derivative as mentioned for the compound (VI).

When the compound (XI) is used in the free acid state, an appropriate condensing agent is preferably used. The same condensing agent as mentioned above may be used in this case, too. When such condensing agent is used, the reaction presumably proceeds via a reactive derivative of the carboxylic acid. The use of a base such as 4-dimethylaminopyridine as a catalyst for the reaction is preferable. The reaction is generally conducted in an inert solvent. Examples of the solvent are water, acetone, dioxane, acetonitrile, chloroform, benzene, methylene chloride, ethylene chloride, tetrahydrofuran, ethyl acetate, N,N-dimethylformamide, pyridine and other organic solvents, and mixtures of these.

The compound (X) can be produced by reacting the compound (II) with the compound (IX) or by reacting the compound (VII) with a compound of the general formula

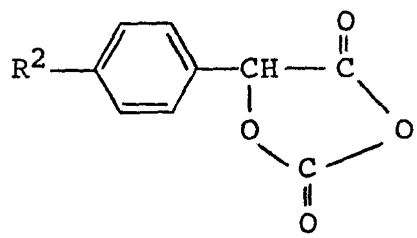

(XII)

wherein $R^2$ is as defined above.

Referring to the former reaction, the compound (II) is preferably submitted to the reaction in the form of a reactive derivative thereof (e.g. alkali metal salt, such as sodium salt or potassium salt, alkaline earth metal salt, such as calcium salt, organic amine salt, such as triethylamine salt or pyridine salt).

The above reactions are preferably conducted with cooling so that the formation of byproduct $\Delta^2$ isomers can be avoided. The reactions can proceed readily in the presence of a solvent which will not adversely affect said reactions (for example, N,N-dimethylformamide, N,N-dimethylacetamide, hexamethylphosphoramide, acetone, acetonitrile, dimethyl sulfoxide, chloroform or methylene chloride).

(Method 3)

A compound of the general formula

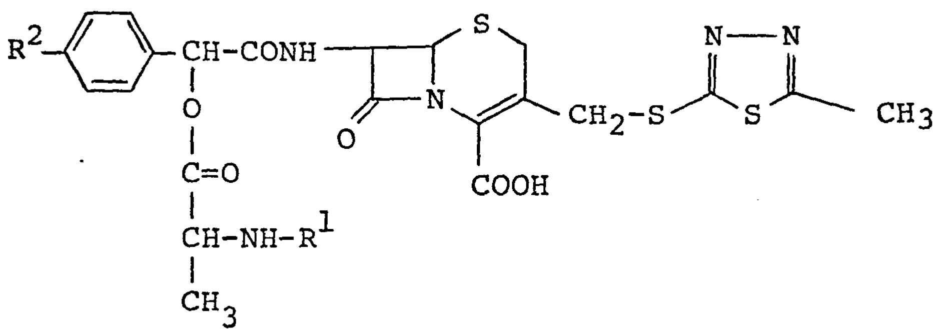

(XIII)

wherein $R^1$ and $R^2$ are as defined above, or a reactive derivative thereof is reacted with the compound (IX).

Referring to the above reaction, the compound (XIII) is submitted to the reaction either in the form of the free carboxylic acid itself or in the form of a reactive derivative of the same sort as mentioned for the compound (VI).

When the compound (IX) is used in the state in which X is hydroxy, a condensing agent as mentioned above is preferably used. When such condensing agent is used, the reaction presumably proceeds via a reactive derivative of the carboxylic acid. It is preferable to use a base, such as 4-dimethylaminopyridine, as a catalyst in carrying out the reaction.

The compound (XIII) can be produced by reacting 7-amino-3-(5-methyl-1,3,4-thiadiazol-2-yl)thiomethyl-3-cephem-4-carboxylic acid (7-ACTD) with the compound (VI).

The compound (VI) is submitted to said acylation reaction preferably in the form of a reactive derivative such as mentioned hereinabove.

The cephalosporin derivatives (I) can be converted to pharmaceutically acceptable acid addition salts, such as mineral acid salts (e.g. hydrochloride), by conventional means.

The cephalosporin derivatives (I) and salts thereof can be isolated and purified by a conventional method.

The cephalosporin derivatives (I) thus produced can

be diluted with excipients or vehicles by *per se* known means to give therapeutic agents for oral administration for the treatment of bacterial infections. The dilution is effected by *per se* known means, such as mixing or blending. Examples of the excipients or vehicles are starch, anhydrous lactose, sugar, calcium carbonate and potassium phosphate.

In said therapeutic agents for oral administration for the treatment of bacterial infections, there may further be incorporated other additives as desired. Preferred examples are binders (e.g. starch, gum arabic, carboxymethylcellulose, hydroxypropylcellulose, crystalline cellulose), lubricants (e.g. magnesium stearate, talc) and disintegrants (e.g. carboxymethylcellulose calcium, talc). After mixing of the components, the mixture itself can be made up into dosage forms suited for oral administration, such as capsules, tablets, fine granules, granules and dry syrups, by *per se* known means.

The dose of the cephalosporin derivative (I) or a salt thereof varies depending on the subject of administration, symptoms and other factors. For treating purulent or suppurative diseases in human adults, for instance, it is administered orally in a single dose of, for example, about 1 to 40 mg/kg of body weight once to four times a day.

The following examples illustrate the invention in further detail but are by no means limitative thereof.

In the examples, Boc is t-butoxycarbonyl.

Example 1

Synthesis of (5-methyl-1,3-dioxol-2-on-4-yl)methyl 7-[D-O-(L-alanyl)mandelamido]-3-(5-methyl-1,3,4-thiadiazol-2-yl)thiomethyl-3-cephem-4-carboxylate hydrochloride

Process (1): (5-Methyl-1,3-dioxol-2-on-4-yl)methyl 7-amino-3-(5-methyl-1,3,4-thiadiazol-2-yl)thiomethyl-3-cephem-4-carboxylate (1.5 g) is dissolved in 45 ml of methylene chloride. At 0°C, there is added 0.92 g of D-O-(L-alanyl)mandelic chloride hydrochloride over 30 minutes, followed by stirring overnight at room temperature. The solid matter is collected by filtration and washed with methylene chloride to give 2.1 g of the title compound in a crude state. Recrystallization from ethanol gives 1.7 g of the title compound.

Process (2): (5-Methyl-1,3-dioxol-2-on-4-yl)methyl 7-[D-O-(N-Boc-L-alanyl)mandelamido]-3-(5-methyl-1,3,4-thiadiazol-2-yl)thiomethyl-3-cephem-4-carboxylate (72 g) is dissolved in 700 ml of ethyl acetate. At -5°C, 7 g of gaseous hydrogen chloride is blown into the solution, followed by stirring at 0°C for 30 minutes. The resulting solid matter is filtered off and washed with isopropyl ether to give 66 g of the title compound in a crude state. Recrystallization from ethanol gives 56 g of the title compound.

Process (3): (5-Methyl-1,3-dioxol-2-on-4-yl)methyl 7-[D-O-(N-formyl-L-alanyl)mandelamido]-3-(5-methyl-1,3,4-thiadiazol-2-yl)thiomethyl-3-cephem-4-carboxylate (2 g) is

dissolved in 50 ml of methanol, followed by addition of 1.32 ml of concentrated hydrochloric acid. The mixture is stirred overnight at room temperature. The reaction mixture is poured into isopropyl ether, and the resulting solid precipitate is filtered off and washed with isopropyl ether to give 1.7 g of the title compound in a crude state. Recrystallization from ethanol gives 1.2 g of the title compound.

Process (4): (5-Methyl-1,3-dioxol-2-on-4-yl)methyl 7-[D-O-[N-(1-methyl-2-ethoxycarbonylvinyl)-L-alanyl]mandelamido]-3-(5-methyl-1,3,4-thiadiazol-2-yl)thiomethyl-3-cephem-4-carboxylate (5 g) is dissolved in 30 ml of methanol, followed by addition, at 0°C, of 20 ml of 4 N hydrogen chloride-in-ethanol solution. The mixture is then stirred at room temperature for 30 minutes. Deithyl ether is added to the reaction mixture, and the solid precipitate is filtered off and washed with diethyl ether to give 3.8 g of the title compound in a crude state. Recrystallization from ethanol gives 2.9 g of the title compound. Melting point: 158°C (decomposition).

IR (nujol, $cm^{-1}$); 1815, 1760, 1690

NMR ($(CD_3)_2SO$, δ value);

1.48 (d, 3H, J=7 Hz, $-\overset{|}{C}H-C\underline{H}_3$)

2.18 (s, 3H, dioxole $-CH_3$)

2.66 (s, 3H, thiadiazole $-CH_3$)

3.62 (br. s, 2H, 2-position $-H_2$)

4.2 (m, 1H, $-\overset{|}{C}\underline{H}-CH_3$)

4.07, 4.65 (d, d, 2H, J=14 Hz, 3-position $-CH_2S-$)

5.05 (d, 1H, J=5 Hz, 6-position -H)

5.15 (s, 2H, dioxole $-CH_2-$)

5.72 (d x d, 1H, J=5 and 9 Hz, 7-position -H)

6.13 (s, 1H, -C<u>H</u>-CONH-)

7.4 (m, 5H, phenyl)

8.73 (br. 3H, $-NH_3^+$)

9.46 (d, 1H, J=9 Hz, -CONH-)

Example 2

Synthesis of (5-methyl-1,3-dioxol-2-on-4-yl)methyl 7-[D-α-(L-alanyloxy)-p-hydroxyphenylacetamido]-3-(5-methyl-1,3,4-thiadiazol-2-yl)thiomethyl-3-cephem-4-carboxylate hydrochloride

(5-Methyl-1,3-dioxol-2-on-4-yl)methyl 7-[D-α-(Boc-L-alanyloxy)-p-hydroxyphenylacetamido]-3-(5-methyl-1,3,4-thiadiazol-2-yl)thiomethyl-3-cephem-4-carboxylate (0.8 g) is dissolved in 3.9 ml of formic acid, followed by addition, at 0°C, of 1.26 ml of 4.08 N hydrogen chloride-in-isopropanol solution. The mixture is then stirred at room temperature for 10 minutes. Diethyl ether is added to the reaction mixture, the solid precipitate is filtered off, washed with diethyl ether and dried to give 586 mg of the title compound.

IR (nujol, $cm^{-1}$); 1830, 1790, 1760, 1695

NMR ($(CD_3)_2SO$, δ value);

1.45 (d, 3H, J=7 Hz, $-CH-CH_3$)

2.18 (s, 3H, dioxole $-CH_3$)

2.66 (s, 3H, thiadiazole $-CH_3$)

3.64 (br. s, 2H, 2-position $-H_2$)

4.08, 4.66 (d, d, J=14 Hz, 3-position $-CH_2S-$)

4 - 4.03 (m, 1H, $-\overset{|}{C}\underline{H}-CH_3$)

5.12 (d, 1H, J=5 Hz, 6-position -H)

5.14 (s, 2H, dioxole $-CH_2-$)

5.69 (d x d, 1H, J=5 and 9 Hz, 7-position -H)

6.0 (s, 1H, $-\overset{|}{C}\underline{H}-CONH-$)

6.78, 7.31 (d, d, 4H, J=8 Hz, phenyl)

8.7 (br. 4H, -OH, $-NH_3^+$)

9.3 (d, 1H, J=9 Hz, -CONH-)

Example 3

(5-Methyl-1,3-dioxol-2-on-4-yl)methyl 7-[D-O-[N-(1-methyl-2-ethoxycarbonylvinyl)-L-alanyl]mandelamido]-3-(5-methyl-1,3,4-thiadiazol-2-yl)thiomethyl-3-cephem-4-carboxylate was treated in the same manner as in process (4) of Example 1 to give the same product as that of Example 1.

Example 4

(5-Methyl-1,3-dioxol-2-on-4-yl)methyl 7-[D-O-(N-trityl-L-alanyl)mandelamido]-3-(5-methyl-1,3,4-thiadiazol-2-yl)thiomethyl-3-cephem-4-carboxylate was treated in the same manner as in Example 2 to give the same product as that of Example 1.

Example 5

(5-Methyl-1,3-dioxol-2-on-4-yl)methyl 7-[D-O-(N-p-methoxybenzyloxycarbonyl-L-alanyl)mandelamido]-3-(5-methyl-1,3,4-thiadiazol-2-yl)thiomethyl-3-cephem-4-carboxylate was treated in the same manner as in process (4) of Example 1 to give the same product as that of Example 1.

Example 6

(5-Methyl-1,3-dioxol-2-on-4-yl)methyl 7-[D-O-(N-trimethylsilyl-L-alanyl)mandelamido]-3-(5-methyl-1,3,4-thiadiazol-2-yl)thiomethyl-3-cephem-4-carboxylate was treated in the same manner as in process (3) of Example 1 to give the same product as that of Example 1.

Example 7

(5-Methyl-1,3-dioxol-2-on-4-yl)methyl 7-[D-O-(N-benzyloxycarbonyl-L-alanyl)mandelamido]-3-(5-methyl-1,3,4-thiadiazol-2-yl)thiomethyl-3-cephem-4-carboxylate was debenzylated with acetic acid-hydrobromic acid mixture, followed by addition of ether. The thus-obtained hydrobromide was converted to the free base and further to the hydrochloride to give the same product as that of Example 1.

Example 8

(5-Methyl-1,3-dioxol-2-on-4-yl)methyl 7-[D-O-(N-2,2,2-trichloroethoxycarbonyl-L-alanyl)mandelamido]-3-(5-methyl-1,3,4-thiadiazol-2-yl)thiomethyl-3-cephem-4-carboxylate is dissolved in 10 ml of acetic acid, followed by addition, at 20°C, of 2 g of activated zinc. The mixture is stirred for 1 hour. Methylene chloride is added, the insoluble matter is filtered off, the filtrate is washed quickly with cold aqueous sodium bicarbonate and cold aqueous sodium chloride

and dried over anhydrous sodium sulfate, the solvent is distilled off under reduced pressure, 0.2 ml of 5 N hydrogen chloride-in-methanol solution is added and, further, 1 ml of methanol and 10 ml of acetone are added to give the compound of Example 1.

Reference Example 1

Production of D-O-(L-alanyl)mandelic chloride hydrochloride

D-O-(L-Alanyl)mandelic acid hydrochloride (7g) is suspended in 140 ml of methylene chloride. With cooling, 140 ml of 8% phosphorus pentaoxide solution in methylene chloride is added. After overnight stirring, the solid is filtered off, washed with methylene chloride and dried to give 7.5 g (94% yield) of the title compound. $[\alpha]_D^{28}$=-56.28° (c=1, dioxane)

IR (nujol, $cm^{-1}$); 3020, 1790, 1750

NMR ($CF_3COOD$, $\delta$ value);

1.83 (d, 3H, J=7 Hz, $-\overset{|}{C}H-C\underline{H}_3$)

4.65 (q, 1H, J=7 Hz, $-\overset{|}{C}\underline{H}-CH_3$)

6.32 (s, 1H, $-\overset{|}{C}\underline{H}-CONH-$)

7.51 (s, 5H, phenyl)

Reference Example 2

Synthesis of D-O-(Boc-L-alanyl)mandelic acid

Boc-L-alanine (2 g) is dissolved in 30 ml of anhydrous tetrahydrofuran. Triethylamine (1.1 g) is added and then a solution of ethyl chloroformate in 10 ml of anhydrous

tetrahydrofuran is added dropwise at -20°C. The mixture is stirred at the same temperature for 30 minutes. To this are added 1.61 g of D-mandelic acid and 50 mg of 4-dimethylaminopyridine, and the mixture is stirred at room temperature for 2 hours. The solvent in distilled off under reduced pressure, ethyl acetate is added for dissolution of the residue, the solution is washed with water and aqueous sodium chloride and dried over anhydrous sodium sulfate, the solvent is then distilled off under reduced pressure, hexane is added, and the mixture is allowed to stand. Thus is obtained 830 mg of the title compound (the same compound as that of Example 2). (Yield 24%)

IR (nujol, $cm^{-1}$); 3500, 3425, 1725, 1690

NMR ($CDCl_3$, δ value);

1.38 (s, 9H, $-C(CH_3)_3$)

1.40 (d, 3H, J=7 Hz, $C\underline{H_3}-\overset{|}{C}H-$)

4.40 (m, 1H, $CH_3-\overset{|}{C}\underline{H}-$)

6.00 (s, 1H, $\phi-\overset{|}{C}\underline{H}-$)

7.2 - 7.5 (m, 6H, ϕ, -COOH)

9.27 (br. s, 1H, -NH-)

Reference Example 3

D-O-(N-Boc-L-alanyl)mandelic acid (49.7 g) as obtained in Reference Example 2 and 62 g of (5-methyl-1,3-dioxol-2-on-4-yl)methyl 7-amino-3-(5-methyl-1,3,4-thiadiazol-2-yl)thiomethyl-3-cephem-4-carboxylate are

dissolved in 600 ml of methylene chloride. At -5°C, there is added dropwise 80 ml of 2 N-N,N'-dicyclohexylcarbodiimide solution in methylene chloride, followed by stirring at the same temperature for 1 hour. The insoluble matter is filtered off, the filtrate is concentrated under reduced pressure, ethyl acetate is added to the residue, again the insoluble matter is filtered off, the filtrate is concentrated under reduced pressure, methanol is added to the residue, and the resulting crystalline precipitate is collected by filtration and dried to give 72 g of (5-methyl-1,3-dioxol-2-on-4-yl)methyl 7-[D-O-(N-Boc-L-alanyl)-mandelamido]-3-(5-methyl-1,3,4-thiadiazol-2-yl)thiomethyl-3-cephem-4-carboxylate. Melting point: 156°C (decomposition)

IR (nujol, $cm^{-1}$); 3340, 1820, 1780, 1750, 1680

NMR ($CDCl_3$, δ value);

1.40 (d, 3H, J=7 Hz, $-\overset{|}{C}H-C\underline{H}_3$)

1.41 (s, 9H, $-C(CH_3)_3$)

2.22 (s, 3H, dioxole $-CH_3$)

2.73 (s, 3H, thiadiazole $-CH_3$)

3.42, 3.82 (d, d, 2H, J=17 Hz, 2-position $-H_2$)

3.89, 4.91 (d, d, 2H, J=14 Hz, 3-position $-CH_2S-$)

4.1 - 4.55 (m, 1H, $-\overset{|}{C}\underline{H}-CH_3$)

5.0 (br. 1H, -NH-)

5.03 (d, 1H, J=5 Hz, 6-position -H)

5.05 (s, 2H, dioxole $-CH_2-$)

5.69 (d x d, 1H, J=5 and 9 Hz, 7-position -H)

6.19 (s, 1H, -CH-CONH-)

7.4 (s, 5H, phenyl)

7.68 (d, 1H, J=9 Hz, -CONH-)

Reference Example 4

(5-Methyl-1,3-dioxol-2-on-4-yl)methyl 7-amino-3-(5-methyl-1,3,4-thiadiazol-2-yl)thiomethyl-3-cephem-4-carboxylate (45.6 g) is dissolved in 500 ml of methylene chloride, followed by addition of 17.8 g of anhydro-O-carboxymandelic acid at 0°C over 15 minutes. The mixture is then stirred at the same temperature for 2 hours. The solvent is then distilled off under reduced pressure, the residue is dissolved in ethyl acetate, the solution is washed with saturated aqueous sodium bicarbonate and saturated aqueous sodium chloride, dried over anhydrous sodium sulfate and concentrated under reduced pressure, and isopropyl ether is added. The resulting solid precipitate is filtered off and recrystallized from methanol to give 49 g of (5-methyl-1,3-dioxol-2-on-4-yl)methyl 7-D-mandelamido-3-(5-methyl-1,3,4-thiadiazol-2-yl)thiomethyl-3-cephem-4-carboxylate. Melting point: 151°C (decomposition).

IR (nujol, $cm^{-1}$); 3350, 1820, 1780, 1730, 1680

NMR ($(CD_3)_2SO$, $\delta$ value);

2.22 (s, 3H, dioxole $-CH_3$)

2.70 (s, 3H, thiadiazole $-CH_3$)

3.59, 3.97 (d, d, 2H, J=17 Hz, 2-position $-H_2$)

4.07, 4.87 (d, d, 2H, J=14 Hz, 3-position $-H_2$)

5.12 (d, 1H, J=5 Hz, 6-position -H)

5.15 (s, 2H, dioxole $-CH_2-$)

5.21 (d, 1H, J=6 Hz, -CH-OH)

5.41 (d, 1H, J=6 Hz, -OH)

5.81 (d x d, 1H, J=5 and 9 Hz, 7-position -H)

7.42 (m, 5H, phenyl)

7.99 (d, 1H, J=9 Hz, -CONH-)

Reference Example 5

(1) To 1.7 g of 7-[D-O-(L-alanyl)mandelamido]-3-(5-methyl-1,3,4-thiadiazol-2-yl)thiomethyl-3-cephem-4-carboxylate and 0.4 g of ethyl acetoacetate, there is added 20 ml of methanol. To the suspension is added, with stirring at room temperature, a solution of 0.2 g of potassium hydroxide in 15 ml of methanol, followed by stirring for 4 hours. To the reaction mixture is added 350 ml of diethyl ether, and the solid precipitate is filtered off and dried to give 1.92 g of potassium 7-[D-O-{N-1-methyl-2-ethoxycarbonylvinyl)-L-alanyl}-mandelamido]-3-(5-methyl-1,3,4-thiadiazol-2-yl)thiomethyl-3-cephem-4-carboxylate.

NMR ($(CD_3)_2SO$, δ value);

1.15 (t, 3H, J=7 Hz, $-CO_2CH_2CH_3$)

1.41 (d, 3H, J=7 Hz, $-CH-CH_3$)

1.92 (s, 3H, $CH_3-C=$)

2.66 (s, 3H, thiadiazole $-CH_3$)

3.4 (br. s, 2H, 2-position $-H_2$)

3.9 - 4.8 (m, 3H, 3-position $-CH_2S-$, $-\overset{|}{C}\underline{H}-CH_3$)

3.98 (q, 2H, J=7 Hz, $-CO_2-C\underline{H}_2-CH_3$)

4.43 (s, 1H, $-\overset{|}{C}=CH-$)

4.94 (d, 1H, J=5 Hz, 6-position -H)

5.50 (d x d, 1H, J=5 and 9 Hz, 7-position -H)

6.01 (s, 1H, $-\overset{|}{C}\underline{H}-CONH-$)

7.36 (s, 5H, phenyl)

8.67 (d, 1H, J=9 Hz, -CONH-)

9.25 (d, 1H, J=9 Hz, -NH-)

(2) A 1 g portion of the compound obtained above in (1) is dissolved in 20 ml of N,N-dimethylformamide. Thereto is added dropwise at -10°C a solution of 0.37 g of 4-bromomethyl-5-methyl-1,3-dioxol-2-one in 2 ml of N,N-dimethylformamide. After stirring at the same temperature for 30 minutes, 100 ml of water is added to the reaction mixture, followed by extraction with two 50 ml portions of ethyl acetate. The ethyl acetate layers are combined, washed with water and saturated aqueous sodium chloride, and dried over anhydrous sodium sulfate. The solvent is distilled off under reduced pressure, diethyl ether is added to the residue, and the resulting solid precipitate is filtered off and dried to give 0.6 g of (5-methyl-1,3-dioxol-2-on-4-yl)methyl 7-[D-O-{N-1-methyl-2-ethoxycarbonylvinyl)-L-alanyl}mandelamido]-3-(5-methyl-1,3,4-thiadiazol-2-yl)thiomethyl-3-cephem-4-carboxylate.

NMR ($(CD_3)_2SO$, δ value);

1.16 (t, 3H, J=7 Hz, $-CO_2-CH_2-C\underline{H}_3$)

1.41 (d, 3H, J=7 Hz, $-\overset{|}{C}H-C\underline{H}_3$)

1.92 (s, 3H, $CH_3-\overset{|}{C}=$)

2.18 (s, 3H, dioxole $-CH_3$)

2.65 (s, 3H, thiadiazole $-CH_3$)

3.65 (br. s, 2H, 2-position $-H_2$)

4.0 (q, 2H, J=7 Hz, $-CO_2C\underline{H}_2CH_3$)

4.0 - 4.8 (m, 3H, 3-position $-CH_2S-$, $-\overset{|}{C}\underline{H}-CH_3$)

4.42 (s, 1H, $-\overset{|}{C}=CH-$)

5.10 (d, 1H, J=5Hz, 6-position -H)

5.13 (s, 2H, dioxole $-CH_2-$)

5.72 (d x d, 1H, J=5 and 9 Hz, 7-position -H)

5.98 (s, 1H, $-\overset{|}{C}\underline{H}-CONH-$)

7.38 (s, 5H, phenyl)

8.70 (d, 1H, J=9 Hz, -CONH-)

9.35 (d, 1H, J=9 Hz, -NH-)

Reference Example 6

To 0.88 g of D-α-(N-Boc-L-alanyl)-p-hydroxyphenylacetic acid and 1 g of (5-methyl-1,3-dioxol-2-on-4-yl)methyl 7-amino-3-(5-methyl-1,3,4-thiadiazol-2-yl)thiomethyl-3-cephem-4-carboxylate, there are added 30 ml of methylene chloride and 15 ml of ethyl acetate. To the suspension, there is added dropwise with stirring at -5°C 2.8 ml of 1N-N,N'-dicyclohexylcarbodiimide in methylene chloride, followed by stirring at the same temperature for 1 hour.

The insoluble matter is filtered off, the filtrate is concentrated under reduced pressure, ethyl acetate is added to the residue, again the insoluble matter is filtered off, isopropyl ether is added to the filtrate, and the resulting solid precipitate is filtered off and dried to give 1.45 g of (5-methyl-1,3-dioxol-2-on-4-yl)methyl 7-[D-α-(N-Boc-L-alanyloxy)-p-hydroxyphenylacetamido]-3-(5-methyl-1,3,4-thiadiazol-2-yl)thiomethyl-3-cephem-4-carboxylate.

NMR ($CDCl_3$, δ value);

1.39 (s, 9H, $-C(CH_3)_3$)

1.40 (d, 3H, J=7 Hz, $-\overset{|}{C}H-C\underline{H}_3$)

2.19 (s, 3H, dioxole $-CH_3$)

2.70 (s, 3H, thiadiazole $-CH_3$)

3.38, 3.80 (d, d, 2H, J=17 Hz, 2-position $-H_2$)

3.89, 4.90 (d, d, 2H, J=14 Hz, 3-position $-CH_2S-$)

4.0 - 4.4 (m, 1H, $-\overset{|}{C}\underline{H}-CH_3$)

4.95 (d, 1H, J=5 Hz, 6-position -H)

5.05 - 5.25 (m, 3H, -NH-, dioxole $-CH_2-$)

5.70 (d x d, 1H, J=5 and 9 Hz, 7-position -H)

6.06 (s, 1H, $-\overset{|}{C}\underline{H}-CONH-$)

6.75, 7.23 (d, d, 4H, J=8 Hz, phenyl)

7.25 (br. 1H, -OH)

7.75 (d, 1H, J=9 Hz, -CONH-)

CLAIM

A method of producing cephalosporin derivatives of the general formula

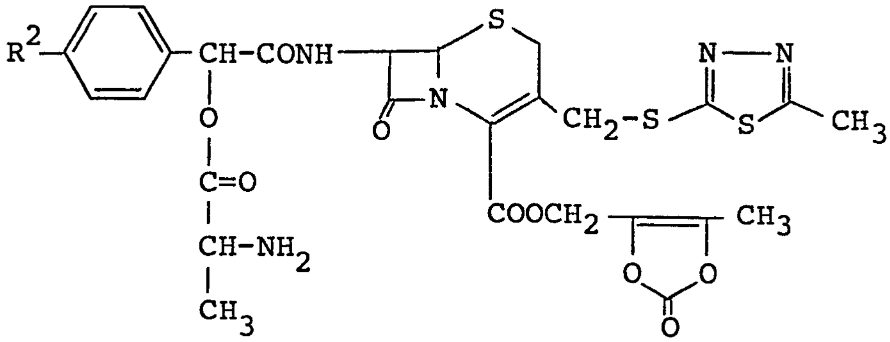

wherein $R^2$ is a hydrogen atom or a hydroxy group, which comprises reacting a compound of the general formula

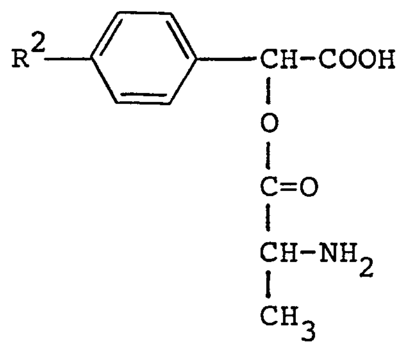

wherein $R^2$ is as defined above, or a reactive derivative thereof with a compound of the general formula

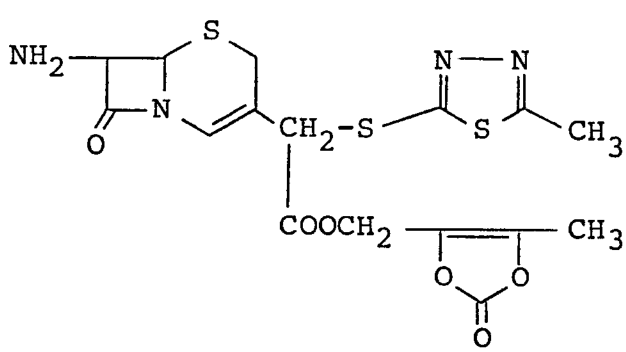

or subjecting a compound of the general formula

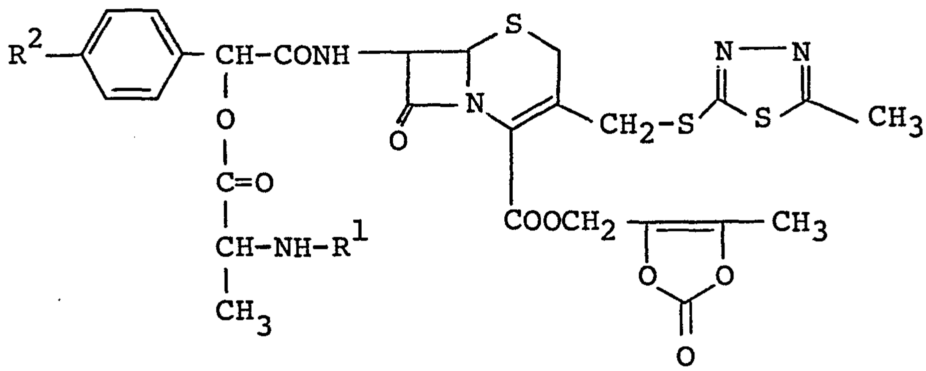

wherein $R^1$ is formyl, t-butoxycarbonyl, 1-methyl-2-methoxycarbonylvinyl, 1-methyl-2-ethoxycarbonylvinyl, benzyloxycarbonyl, p-methoxybenzyloxycarbonyl, chloroacetyl, trityl, silyl, or 2,2,2-trichloroethoxycarbonyl and $R^2$ is as defined above, to $R^1$ elimination reaction.

0186706

# INTERNATIONAL SEARCH REPORT

International Application No. PCT/JP 84/00267

**I. CLASSIFICATION OF SUBJECT MATTER** (if several classification symbols apply, indicate all) [3]

According to International Patent Classification (IPC) or to both National Classification and IPC

Int. $Cl^3$ C07D501/04, 501/06

**II. FIELDS SEARCHED**

Minimum Documentation Searched [4]

| Classification System | Classification Symbols |
|---|---|
| I P C | C07D501/04, 501/06 |

Documentation Searched other than Minimum Documentation to the Extent that such Documents are Included in the Fields Searched [5]

**III. DOCUMENTS CONSIDERED TO BE RELEVANT** [14]

| Category* | Citation of Document, [16] with indication, where appropriate, of the relevant passages [17] | Relevant to Claim No. [18] |
|---|---|---|
| | | |

* Special categories of cited documents: [15]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search [2] | Date of Mailing of this International Search Report [2] |
|---|---|
| July 30, 1984 (30.07.84) | August 6, 1984 (06.08.84) |
| **International Searching Authority [1]** | **Signature of Authorized Officer [20]** |
| Japanese Patent Office | |